(19) Europäisches Patentamt
European Patent Office
Office européen des brevets

(11) Publication number: **0 012 570**
**B1**

(12) **EUROPEAN PATENT SPECIFICATION**

(45) Date of publication of patent specification: **12.10.83**

(21) Application number: **79302805.1**

(22) Date of filing: **06.12.79**

(51) Int. Cl.³: **C 07 C 15/02,**
C 07 C 7/148,
C 07 B 29/00,
B 01 J 29/28, B 01 J 29/36
//C07C39/06, C07C37/86,
C07C47/542, C07C45/85,
C07C25/02, C07C17/38,
C07C87/50, C07C85/26

(54) **Selective reaction of 1,4-disubstituted benzene compounds.**

(30) Priority: **14.12.78 US 969745**
**14.12.78 US 969744**

(43) Date of publication of application:
**25.06.80 Bulletin 80/13**

(45) Publication of the grant of the patent:
**12.10.83 Bulletin 83/41**

(84) Designated Contracting States:
**BE DE FR GB IT NL**

(56) References cited:
**DE - A - 2 558 035**
**GB - A - 809 908**
**NL - A - 7 202 547**
**US - A - 2 840 621**

(73) Proprietor: **MOBIL OIL CORPORATION**
**150 East 42nd Street**
**New York New York 10017 (US)**

(72) Inventor: **Wu, Margaret May-Som**
**7 Warenton Way, Belle Mead**
**New Jersey 08502, Somerset (US)**
Inventor: **Young, Lewis Bewster**
**Pineview Court, Skillman**
**New Jersey 08558, Somerset (US)**

(74) Representative: **West, Alan Harry**
**Mobil Court 3 Clements Inn**
**London WC2A 2EB (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

Selective reaction of 1,4-disubstituted benzene compounds

This invention relates to a process for selectively reacting and removing 1—4 disubstituted benzene compounds containing not more than one methyl substituent from mixtures containing such compounds by reacting in the presence of a specified shape selective crystalline zeolite catalyst.

The separation of *meta* or *ortho* isomers from *para* disubstituted aromatic compounds is a difficult but necessary step in the production and isolation of *ortho* and *meta* compounds. The approach has most frequently been to take advantage of the differences in the boiling points of the various isomers and utilize fractional distillation to make the separation. However, as can be seen from Table IA, the differences between the temperatures at which the isomers boil are in reality so very small that in order to achieve efficient separation one must employ very elaborate and expensive distillation columns.

TABLE IA
Boiling points at 760 mm Hg

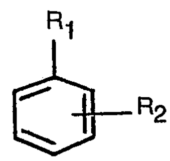

| $R_1$ | $R_2$ | Isomer ortho | meta | para |
|---|---|---|---|---|
| $CH_3$— | $CH_3$— | 144.4°C | 139.1°C | 138.4°C |
| $CH_3$— | $CH_3CH_2$— | 165.2°C | 161.3°C | 162.0° |
| $CH_3$— | 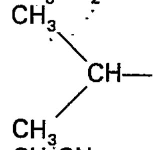 | 178.3°C | 175.1°C | 177.1°C |
| $CH_3CH_2$— | $CH_3CH_2$— | 183.5°C | 181.0°C | 183.8°C |
| 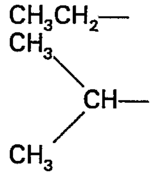 | 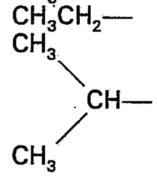 | 210.0°C | 203.2°C | 210.3°C |

The time-honored method of isolating or removing particular positional isomers from isomeric mixtures of disubstituted aromatic compounds has also been the classical fractional distillation technique. This method, however, has not been very practical on an industrial scale unless there is a significant difference in the boiling points of the various isomers. In terms of many important industrial aromatic compounds, particularly those having one or more polar substituents, separation or significant enrichment of particular isomers by boiling point is an extremely difficult and expensive endeavor, and at times is quite impossible. Table IB below will illustrate the problem with the boiling points of a few typical compounds.

TABLE IB

| Name | B.P., °C ortho | meta | para |
|---|---|---|---|
| Hydroxy isopropyl benzene (isopropylphenol) (cumenol) | 213.4[a] | 228[a] | 228—30[b] |
| Amino isopropyl benzene (isopropylaniline) | 220—1[b] | | 225[a] |
| Chloro isopropyl benzene (chlorocumene) | 191[c] | 65[7mm] 62—68[8mm] | 198.3[a] |
| Methyl benzaldehyde (tolualdehyde) | 220[a] | 199[a] | 204—5[a] |
| Chlorobenzaldehyde | 211.9[a] | 213—4[a] | 213—4[a] |

[a] 760 mm Hg [b] 745 mm Hg [c] 745.6 mm Hg

Dutch Patent Application 7202547 describes a process for separating meta-t.butylphenol from mixed *m* and *p* isomers using acid activated clay catalysts in the presence of an acid and an alkylatable aromatic hydrocarbon; and British Patent 809908 describes the synthesis of meta-diisopropylbenzene by a process that includes the step of separating the *m* and *p* isomers over a silicate cracking catalyst. Both processes, however, yield results inferior to those of the process of this invention due to the non-crystalline nature of the catalysts used in them.

U.S. Patent No. 3,029,300 to Schaeffer discloses a selective clathration process for the separation of xylene isomers, but this also involves an elaborate procedure requiring very specialized and expensive equipment.

A catalytic process for the selective production of particular xylene isomers, involving a platinum on alumina catalyst, is disclosed in U.S. Patent No. 3,078,318 to Berger.

Selective production of *para* dialkylbenzenes in the presence of specific zeolite catalysts is described in U.S. Patents No. 3,965,209 to Butter et al; 4,001,346 to Chu; 4,086,287 to Kaeding et al and 4,090,981 to Rodewald.

It has now been discovered that both polar and non-polar 1,2-disubstituted and 1,3-disubstituted aromatic compounds containing not more than one methyl substituent may be conveniently, efficiently and economically recovered on an industrial scale by subjecting mixtures of such compounds containing undesirable 1,4-isomers thereof to treatment with a particular type of crystalline zeolite catalyst. The 1,4-disubstituted isomer is selectively reacted (dealkylated) to give products with significantly lower boiling points to permit a conventional, inexpensive separation thereof, leaving the 1,2- and/or 1,3-disubstituted isomer in excess of equilibrium. Following the teachings of this invention, 1,2-disubstituted and/or 1,3-disubstituted aromatics may be selectively produced either as the sole isomers or as the major isomers of the desired disubstituted aromatic compound in admixture with a minor amount of the 1,4-disubstituted isomer thereof.

The process of the invention involves contacting an isomeric mixture of disubstituted aromatic compounds, containing not more than one methyl substituent under conversion conditions, with a specific type of shape selective crystalline zeolite catalyst, whereupon the 1,4-disubstituted isomer is selectively cracked or transalkylated leaving the product enriched in 1,2- and/or 1,3-disubstituted isomer.

The selective reaction of the 1,4-disubstituted isomer in the presence of the specified catalyst is conducted at a temperature of between 150°C and 800°C, and preferably within the range of 250°C to 550°C. The zeolite catalyst utilized herein is characterized by a silica to alumina ratio of at least about 12 and a constraint index, as hereinafter defined, within the range of 1 to 12.

The disubstituted aromatic compounds of interest in the process of this invention comprise those defined by the generic formula:

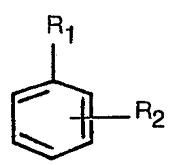

wherein $R_1$ is a $C_1$ to $C_8$ alkyl, alkylene or alkyne group; or a polar substituent such as: amino, alkylamino, aldehyde, oxy, carboxy, carbonyl, alkoxy, carboxylate, halogen, mono-, di- or trihaloalkyl, alkylester, hydroxy, hydroperoxy, thiol, sulfinyl, sulfonyl, alkylthio, imino, hydroxylimino, nitroso, nitro, cyano, cyanato, thiocyanato, carbamate, and $R_2$ is a $C_1$ to $C_8$ alkyl, alkylene or alkyne a polar group the same as or different from $R_1$. $R_1$ and $R_2$ can be the same group or different when both $R_1$ and $R_2$ are alkyl, alkylene or alkyne at least one must be greater than methyl.

In accordance with the present invention, mixtures comprising positional isomers of one or more of such polar or non-polar disubstituted aromatic compounds, said isomers being the 1,2-isomer and/or the 1,3-isomer with at least some of the 1,4-isomer present, are brought into contact, under cracking or transalkylation conditions, with a bed comprising a particulare catalyst containing a crystalline zeolite as hereinafter defined. The 1,4-disubstituted isomer is selectively removed from the mixture in its entirety or at least in substantial part, by carrying out the process at temperatures of between 150°C and 800°C, pressures of between $10^4$ and about $10^7 N/m^2$ (0.1 to 100 atmospheres), and a feed weight hourly space velocity (WHSV) of between 0.1 and 100. The latter WHSV is based upon the weight of the catalyst compositions, i.e. the total weight of active catalyst and binder therefor. It is preferred that contact between the catalyst and the disubstituted aromatic compounds be carried out at from 250°C to 550°C, and at a WHSV of from 0.2 to 50. Although the reaction normally takes place at atmospheric pressure (i.e. $10^5 N/m^2$) the preferred pressure range extends from $2 \times 10^4$ to $2.5 \times 10^6 N/m^2$ (0.2 to 25 atmospheres). The 1,2-disubstituted aromatic compounds and/or the 1,3-disubstituted aromatics, singly or together as desired, may subsequently be separated from the reaction effluent by any suitable means.

The process of this invention may be carried out as a batch-type, semi-continuous or continuous operation utilizing a fixed, fluidized or moving bed isomeric mixture of non-polar disubstituted aromatic

compounds, are passed concurrently or countercurrently through a moving fluidized bed of the catalyst. The fluidized catalyst after use is conducted to a regeneration zone wherein coke is burned from the catalyst in an oxygen-containing atmosphere, e.g. air, at an elevated temperature, after which the regenerated catalyst is recycled to the conversion zone for further contact with the aromatic reactants.

The process may be carried out in a system wherein the disubstituted compounds are in either the liquid or the vapor state, and the mixture of disubstituted aromatic compounds may be substantially pure (i.e. contain no substantial quantity of hydrocarbon material other than said mixed isomers of said disubstituted aromatic material) or may contain substantial amounts of other hydrocarbon material. The latter situation is such as would exist when the feed stream for the instant process also comprises the effluent stream of an earlier upstream process, for instance a process for the manufacture of disubstituted aromatic compounds. Also, the feed stream for the process of this invention may contain other inert materials as diluents or solvents. Suitable diluents include, but are not limited to: methane, nitrogen, propane, hexane, steam, helium hydrogen, carbon dioxide, organic acids (for example formic and acetic acids) etc.

The crystalline zeolites utilized herein are members of a novel class of zeolites that exhibits unusual properties. Although these zeolites have unusually low alumina contents, i.e. high silica to alumina ratios, they are very active even when the silica to alumina ratio exceeds 30. The activity is surprising since catalytic activity is generally attributed to framework aluminum atoms and/or cations associated with these aluminum atoms. These zeolites retain their crystallinity for long periods in spite of the presence of steam at high temperature which induces irreversible collapse of the framework of other zeolites, e.g. of the X and A type. Furthermore, carbonaceous deposits, when formed, may be removed by controlled burning at higher than usual temperatures to restore activity. These zeolites, used as catalysts, generally have low coke-forming activity and therefore are conducive to long times on streams between regenerations by burning with oxygen-containing gas such as air.

An important characteristic of the crystal structure of this class of zeolites is that it provides constrained access to and egress from the intracrystalline free space by virtue of having an effective pore size intermediate between the small pore Linde A and the large pore Linde X, i.e. the pore windows of the structure have about a size such as would be provided by 10-membered rings of silicon atoms interconnected by oxygen atoms. It is to be understood, of course, that these rings are those formed by the regular disposition of the tetrahedra making up the anionic framework of the crystalline zeolite, the oxygen atoms themselves being bonded to the silicon or aluminum atoms at the centers of the tetrahedra. Briefly, the preferred type zeolites useful in this invention possesses, in combination: a silica to alumina mole ratio of at least 12; and a structure providing constrained access to the intracrystalline free space.

The silica to alumina ratio referred to may be determined by conventional analysis. This ratio is meant to represent, as closely as possible, the ratio in the rigid anionic framework of the zeolite crystal and to exclude aluminum in the binder or in cationic or other form within the channels. Although zeolites with a silica to alumina ratio of at least 12 are useful, it is preferred to use zeolites having higher ratios of at least 30. Such zeolites, after activation, acquire an intracrystalline sorption capacity for normal hexane which is greater than that for water, i.e. they exhibit "hydrophobic" properties. It is believed that this hydrophobic character is advantageous in the present invention.

The zeolites useful in this invention have an effective pore size such as to freely sorb normal hexane. In addition, the structure must provide constrained access to large molecules. It is sometimes possible to judge from a known crystal structure whether such constrained access exists. For example, if the only pore windows in a crystal are formed by 8-membered rings of silicon and aluminum atoms, then access by molecules of larger cross-section than hexane is excluded and the zeolite is not of the desired type. Windows of 10-membered rings are preferred, although in some instances excessive puckering of the rings or pore blockage may render these zeolites ineffective. Twelve-membered rings usually do not offer sufficient constraint to produce the advantageous conversions, although the puckered 12-ring structure of TMA offretite shows constrained access. Other 12-ring structures may exist which, due to pore blockage or to other cause, may be operative.

Rather than attempt to judge from crystal structure whether or not a zeolite possesses the necessary constrained access to molecules larger than normal paraffins, a simple determination of the "Constraint Index" as herein defined may be made by passing continuously a mixture of an equal weight of hexane and 3-methylpentane over a small sample, approximately one gram or less, of the zeolite at atmospheric pressure according to the following procedure. A sample of the zeolite, in the form of pellets or extrudate, is crushed to a particle size about that of coarse sand and mounted in a glass tube. Prior to testing, the zeolite is treated with a stream of air at 540°C for at least 15 minutes. The zeolite is then flushed with helium and the temperature adjusted between 290°C and 510°C to give an overall conversion between 10% and 60%. The mixture of hydrocarbons is passed at 1 liquid hourly space velocity (i.e. 1 volume of liquid hydrocarbon per volume of zeolite per hour) over the zeolite with a helium dilution to give a helium to total hydrocarbon mole ratio of 4:1. After 20 minutes on stream, a sample of the effluent is taken and analyzed, most conveniently by gas chromatography, to determine the fraction remaining unchanged for each of the two hydrocarbons.

The "Constraint Index" is calculated as follows:

$$\text{Constraint Index} = \frac{\log_{10}\text{ (fraction of hexane remaining)}}{\log_{10}\text{ (fraction of 3-methylpentane remaining)}}$$

The Constraint Index approximates the ratio of the cracking rate constants for the two hydrocarbons. Zeolites suitable for the present invention are those having a Constraint Index of 1 to 12. Constraint Index (CI) values for some typical zeolites are:

| Zeolite | C.I. |
|---|---|
| ZSM-5 | 8.3 |
| ZSM-11 | 8.7 |
| ZSM-12 | 2 |
| ZSM-23 | 9.1 |
| ZSM-35 | 4.5 |
| ZSM-38 | 2 |
| TMA Offretite | 3.7 |
| Beta | 0.6 |
| ZSM-4 | 0.5 |
| H-Zeolon (mordenite) | 0.4 |
| REY | 0.4 |
| Amorphous Silica-Alumina | 0.6 |
| Erionite | 38 |

The above-described Constraint Index is an important and even critical definition of those zeolites which are useful in the instant invention. The very nature of this parameter and the recited technique by which it is determined, however, admit of the possibility that a given zeolite can be tested under somewhat different conditions and thereby have different Constraint Indices. Constraint Index seems to vary somewhat with severity of operation (conversion) and the presence or absence of binders. Therefore, it will be appreciated that it may be possible to so select test conditions to establish more than one value in the range of 1 to 12 for the Constraint Index of a particular zeolite. Such a zeolite exhibits the constrained access as herein defined and is to be regarded as having a Constraint Index of 1 to 12. Also contemplated herein as having a Constraint Index of 1 to 12 and therefore within the scope of the novel class of highly siliceous zeolites are those zeolites which, when tested under two or more sets of conditions within the above specified ranges of temperature and conversion, produce a value of the Constraint Index slightly less than 1, e.g. 0.9, or somewhat greater than 12, e.g. 14 or 15, with at least one other value of 1 to 12. Thus, it should be understood that the Constraint Index value as used herein is an inclusive rather than an exclusive value. That is, a zeolite when tested by any combination of conditions within the testing definition set forth hereinabove to have a Constraint Index of 1 to 12 is intended to be included in the instant catalyst definition regardless that the same identical zeolite tested under other defined conditions may give a Constraint Index value outside of 1 to 12.

The class of zeolites defined herein is exemplified by ZSM-5, ZSM-11, ZSM-12, ZSM-23, ZSM-35, ZSM-38, and other similar materials. U.S. Patent 3,702,886 describes ZSM-5.

ZSM-11 is described in U.S. Patent 3,709,979.

ZSM-12 is described in U.S. Patent 3,832,449.

ZSM-23 is described in U.S. Patent 4,076,842.

ZSM-35 is described in U.S. Patent 4,016,245.

ZSM-38 is described in U.S. Patent 4,046,859.

The specific zeolites described, when prepared in the presence of organic cations, are substantially catalytically inactive, possibly because the intracrystalline free space is occupied by organic cations from the forming solution. They may be activated by heating in an inert atmosphere at 540°C for one hour, for example, followed by base exchange with ammonium salts followed by calcination at 540°C in air. The presence of organic cations in the forming solution may not be absolutely essential to the formation of this type zeolite; however, the presence of these cations does appear to favor the formation of this special class of zeolite. More generally, it is desirable to activate this type catalyst by base exchange with ammonium salts followed by calcination in air at 540°C for from 15 minutes to 24 hours.

Natural zeolites may sometimes be converted to this type zeolite catalyst by various activation procedures and other treatments such as base exchange, steaming, alumina extraction und calcination, in combinations. Natural minerals which may be so treated include ferrierite, brewsterite, stilbite, dachiardite, epistilbite, heulandite, and clinoptilolite. The preferred crystalline aluminosilicates for use in this invention are ZSM-5, ZSM-11, ZSM-12, ZSM-23, ZSM35, and ZSM-38, with ZSM-5, ZSM-11 and ZSM-23 being particularly preferred.

In a preferred aspect of this invention, the zeolites hereof are selected as those having a crystal framework density, in the dry hydrogen form, of not less than about 1.6 grams per cubic centimeter. It

has been found that zeolites which satisfy all three of these criteria are most desired for several reasons. When hydrocarbon products or by-products are catalytically formed, for example, such zeolites tend to maximize the production of gasoline boiling range hydrocarbon products. Therefore, the preferred zeolites of this invention are those having a Constraint Index as defined above of 1 to 12, a silica to alumina ratio of at least about 12 and a dried crystal density of not less than 1.6 grams per cubic centimeter. Thy dry density for known structures may be calculated from the number of silicon plus aluminum atoms per 1000 cubic Angstroms, as given, e.g. on Page 19 of the article on Zeolite Structure by W. M. Meier. This paper, the entire contents of which are incorporated herein by reference, is included in "Proceedings of the Conference on Molecular Sieves, London, April 1967," published by the Society of Chemical Industry, London, 1968.

When the crystal structure is unknown, the crystal framework density may be determined by classical pyknometer techniques. For example, it may be determined by immersing the dry hydrogen form of the zeolite in an organic solvent which is not sorbed by the crystal. Or, the crystal density may be determined by mercury porosimetry, since mercury will fill the interstices between crystals but will not penetrate the intracrystalline free space. It is possible that the unusual sustained activity and stability of this class of zeolites is associated with its high crystal anionic framework density of not less than about 1.6 grams per cubic centimeter. This high density must necessarily be associated with a relatively small amount of free space within the crystal, which might be expected to result in more stable structures. This free space, however, is important as the locus of catalytic activity.

Crystal framework densities of some typical zeolites including some which are not within the purview of this invention are:

| Zeolite | Void volume | Framework density |
|---|---|---|
| Ferrierite | 0.28 cc/cc | 1.76 g/cc |
| Mordenite | .28 | 1.7 |
| ZSM-5, -11 | .29 | 1.79 |
| ZSM-12 | | 1.8 |
| ZSM-23 | | 2.0 |
| Dachiardite | .32 | 1.72 |
| L | .32 | 1.61 |
| Clinoptilolite | .34 | 1.71 |
| Laumontite | .34 | 1.77 |
| ZSM-4 (Omega) | .38 | 1.65 |
| Heulandite | .39 | 1.69 |
| P | .41 | 1.57 |
| offretite | .40 | 1.55 |
| Levynite | .40 | 1.54 |
| Erionite | .35 | 1.51 |
| Gmelinite | .44 | 1.46 |
| Chabazite | .47 | 1.45 |
| A | .5 | 1.3 |
| Y | .48 | 1.27 |

When synthesized in the alkali metal form, the zeolite is conveniently converted to the hydrogen form, generally by intermediate formation of the ammonium form as a result of ammonium ion exchange and calcination of the ammonium form to yield the hydrogen form. In addition to the hydrogen form, other forms of the zeolite wherein the original alkali metal has been reduced to less than 1.5 percent by weight may be used. Thus, the original alkali metal of the zeolite may be replaced by ion exchange with other suitable ions of Groups IB to VIII of the Periodic Table, including, by way of example, nickel, copper, zinc, palladium, calcium or rare earth metals.

In practicing the desired conversion process, it may be desirable to incorporate the above described crystalline zeolite in another material resistant to the temperature and other conditions employed in the process. Such matrix materials include synthetic or naturally occurring substances as well as inorganic materials such as clay, silica and/or metal oxides. The latter may be either naturally occurring or in the form of gelatinous precipitates or gels including mixtures of silica and metal oxides. Naturally occurring clays which can be composited with the zeolite include those of the montmorillonite and kaolin families, which families include the sub-bentonites and the kaolins commonly known as Dixie, McNamee-Georgia and Florida clays or others in which the main mineral constituent is halloysite, kaolinite, dickite, nacrite or anauxite. Such clays can be used in the raw state as originally mined or initially subjected to calcination, acid treatment or chemical modification.

In addition to the foregoing materials, the zeolites employed herein may be composited with a porous matrix material, such as alumina, silica-alumina, silica-magnesia, silica-zirconia, silica-thoria, silica-berylia, silica-titania as well as ternary compositions, such as silica-alumina-thoria, silica-alumina-zirconia, silica-alumina-magnesia and silica-magnesia-zirconia. The matrix may be in the form of a cogel. The relative proportions of zeolite component and inorganic oxide gel matrix on an anhydrous basis may vary widely, with the zeolite content ranging from between 1 to 99 percent by weight and more usually in the range of 5 to 80 percent by weight of the dry composite.

The crystalline zeolites employed may be modified prior to use by combining therewith a small amount, generally in the range of 0.5 to 40 weight percent, preferably of a difficulty reducible oxide, such as the oxides of phosphorous, boron, magnesium or combinations thereof and also oxides of antimony. Modification of the zeolite with the desired oxide or oxides can readily be effected by contacting the zeolite with a solution of an appropriate compound of the element to be introduced, followed by drying and calcining to convert the compound to its oxide form.

Representative phosphorus-containing compounds which may be used include derivatives of groups represented by $PX_3$, $RPX_2$, $R_2PX$, $R_3P$, $X_3PO$, $(XO)_3PO$, $(XO)_3P$, $R_3P{=}O$, $R_3P{=}S$, $RPO_2$, $RPS_2$, $RP(O)(OX)_2$, $RP(S)(SX)_2$, $R_2P(O)OX$, $R_2P(S)SX$, $RP(OX)_2$, $RP(SX)_2$, $ROP(OX)_2$, $RSP(SX)_2$, $(RS)_2PSP(SR)_2$, and $(RO)_2POP(OR)_2$, where R is an alkyl or aryl, such as phenyl radical and X is hydrogen, R, or halide. These compounds include primary, $RPH_2$, secondary, $R_2PH$ and tertiary $R_3P$, phosphines such as butyl phosphine; the tertiary phosphine oxides $R_3PO$, such as tributylphosphine oxide, the tertiary phosphine sulfides, $R_3PS$, the primary, $RP(O)(OX)_2$, and secondary, $R_2P(O)OX$, phosphonic acids such as benzene phosphonic acid; the corresponding sulfur derivatives such as $RP(S)(SX)_2$ and $R_2P(S)SX$, the esters of the phosphonic acids such as diethyl phosphonate, $(RO)_2P(O)H$, dialkyl alkyl phosphonates, $(RO)_2P(O)R$, and alkyl dialkylphosphinates, $(RO)P(O)R_2$; phosphinous acids, $R_2POX$, such as diethylphosphinous acid, primary, $(RO)P(OX)_2$, secondary, $(RO)_2POX$, and tertiary; $(RO)_3P$; phosphites; and esters thereof such as the monopropyl ester, alkyl dialkylphosphinites, $(RO)PR_2$, and dialkyl alkylphosphonite, $(RO)_2PR$ esters. Corresponding sulfur derivatives may also be employed including $(RS)_2P(S)H$, $(RS)_2P(S)R$, $(RS)P(S)R_2$, $R_2PSX$, $(RS)P(SX)_2$, $(RS)_2PSX$, $(RS)_3P$, $(RS)PR_2$ and $(RS)_2PR$. Examples of phosphite esters include trimethylphosphite, triethylphosphite, diisopropylphosphite, butylphosphite; and pyrophosphites such as tetraethylpyrophosphite. The alkyl groups in the mentioned compounds contain one to four carbon atoms.

Other suitable phosphorus-containing compounds include the phosphorus halides such as phosphorus trichloride, bromide, and iodide, alkyl phosphorodichloridites, $(RO)PCl_2$, dialkyl phosphorochloridites, $(RO)_2PCl$ dialkylphosphinochloridites, $R_2PCl$, alkyl alkylphosphonochloridates, $(RO)(R)P(O)Cl$, dialkyl phosphinochloridates, $R_2P(O)Cl$ and $RP(O)Cl_2$. Applicable corresponding sulfur derivatives include $(RS)PCl_2$, $(RS)_2PX$, $(RS)(R)P(S)Cl$ and $R_2P(S)Cl$.

Preferred phosphorus-containing compounds include diphenyl phosphine chloride, trimethylphosphite and phosphorus trichloride, phosphoric acid, phenyl phosphine oxychloride, trimethylphosphate, diphenyl phosphinous acid, diphenyl phosphinic acid, diethylchlorothiophosphate, methyl acid phosphate and other alcohol-$P_2O_5$ reaction products.

Reaction of the zeolite with the phosphorus compound is effected by contacting the zeolite with such compound. Where the treating phosphorus compound is a liquid, such compound can be in solution in a solvent at the time contact with the zeolite is effected. Any solvent relatively inert with respect to the treating compound and the zeolite may be employed. Suitable solvents include water and aliphatic, aromatic or alcoholic liquids. Where the phosphorus-containing compound is, for example, trimethylphosphite or liquid phosphorus trichloride, a hydrocarbon solvent such as octane may be employed. The phosphorus-containing compound may be used without a solvent, i.e., may be used as a neat liquid. Where the phosphorus-containing compound is in the gaseous phase, such as where gaseous phosphorus trichloride is employed, the treating compound can be used by itself or can be used in admixture with a gaseous diluent relatively inert to the phosphorus-containing compound and the zeolite, such as air or nitrogen, or with an organic solvent, such as octane or toluene.

Prior to reacting the zeolite with the phosphorus-containing compound, the zeolite may be dried. Drying can be effected in the presence of air. Elevated temperatures may be employed. However, the temperature should not be such that the crystal structure of the zeolite is destroyed.

Heating of the phosphorus-containing catalyst subsequent to preparation and prior to use is also preferred. The heating can be carried out in the presence of oxygen, for example, air. Heating can be at a temperature of 150°C. However, higher temperatures, e.g., up to 500°C, are preferred. Heating is generally carried out for 3—5 hours but may be extended to 24 hours or longer. While heating temperatures above 500°C can be employed, they are generally not necessary. At temperatures of 1000°C the crystal structure of the zeolite tends to deteriorate.

The amount of phosphorus incorporated with the zeolite should be at least 0.25 percent by weight. However, it is preferred that the amount of phosphorus in the zeolite be at least 1 percent by weight when the same is combined with a binder, e.g. 35 weight percent of alumina. The amount of phosphorus can be as high as 25 percent by weight or more depending on the amount and type of binder present. Preferably, the amount of phosphorus added to the zeolite is between 0.5 and 15% percent by weight.

The amount of phosphorus incorporated with the zeolite by reaction with elemental phosphorus or phosphorus-containing compound will depend upon several factors. One of these is the reaction time, i.e., the time that the zeolite and the phosphorus-containing source are maintained in contact

with each other. With greater reaction times, all other factors being equal, a greater amount of phosphorus is incorporated with the zeolite. Other factors upon which the amount of phosphorous incorporated with the zeolite is dependent include reaction temperature, concentration of the treating compound in the reaction mixture, the degree to which the zeolite has been dried prior to reaction with the phosphorus-containing compound, the conditions of drying of the zeolite after reaction of the zeolite with the treating compound, and the amount and type of binder incorporated with the zeolite.

Another suitable modifying oxide is that of magnesium. Representative magnesium-containing compounds include magnesium acetate, magnesium nitrate, magnesium benzoate, magnesium propionate, magnesium 2-ethylhexoate, magnesium carbonate, magnesium formate, magnesium oxylate, magnesium amide, magnesium bromide, magnesium hydride, magnesium lactate, magnesium laurate, magnesium oleate, magnesium palmitate, magnesium salicylate, magnesium stearate and magnesium sulfide.

Reaction of the zeolite with the treating magnesium compound is effected by contacting the zeolite with such compound. Where the treating compound is a liquid, such compound can be in solution in a solvent at the time contact with the zeolite is effected. Any solvent relatively inert with respect to the treating magnesium compound and the zeolite may be employed. Suitable solvents include water and aliphatic, aromatic or alcoholic liquid. The treating compound may also be used without a solvent, i.e. may be used as a neat liquid. Where the treating compound is in the gaseous phase, it can be used by itself or can be used in admixture with a gaseous diluent relatively inert to the treating compound and the zeolite such as helium or nitrogen, or with an organic solvent such as octane or toluene.

Heating of the magnesium compound impregnated catalyst subsequent to preparation and prior to use is preferred. The heating can be carried out in the presence of oxygen, for example, air. Heating can be at a temperature of 150°C. However, higher temperatures, e.g., up to 500°C, are preferred. Heating is generally carried out for 1—5 hours but may be extended to 24 hours or longer. While heating temperatures above 500°C may be employed, they are generally not necessary. At temperatures of 1000°C the crystal structure of the zeolite tends to deteriorate. After heating in air at elevated temperatures, the oxide form of magnesium is present.

The amount of magnesium oxide incorporated in the zeolite should be at least 0.25 percent by weight. However, it is preferred that the amount of magnesium oxide in the zeolite be at least 1 percent by weight, particularly when the same is combined with a binder, e.g., 35 weight percent of alumina. the amount of magnesium oxide can be as high as 25 percent by weight or more depending on the amount and type of binder present. Preferably, the amount of magnesium oxide added to the zeolite is between 0.5 and 15 percent by weight.

Boron oxide is also an effective modifying component. Representative boron-containing compounds include boric acid, trimethylborate, boron hydride, boron oxide, boron sulfide, butylboron dimethoxide, butylboronic acid, dimethylboric anhydric, hexamethylborazine, phenylboric acid, triethylborane, tetramethylammonium borohydride, triphenyl boron, and allylborate.

Reaction of the zeolite with the boron compound is effected by contacting the zeolite with such compound. Where the treating boron compound is a liquid, such compound can be in solution in a solvent at the time contact with the zeolite is effected. Any solvent relatively inert with respect to the treating compound and the zeolite may be employed. Suitable solvents include water and aliphatic, aromatic or alcoholic liquids. Where the boron containing compound is, for example, trimethylborate, a hydrocarbon solvent such as octane may be employed. The boron-containing compound may be used without a solvent, i.e. may be used as neat liquid. Where the boron-containing compound is in the gaseous phase, such as where gaseous diborane is employed, the treating compound can be used by itself or can be used in admixture with a gaseous diluent inert to the boron-containing compound and the zeolite, such as nitrogen or helium, or with an organic solvent, such as octane.

Prior to reacting the zeolite with the boron-containing compound, the zeolite may be dried. Drying can be effected in the presence of air. Elevated temperatures may be employed. However, the temperature should not be such that the crystal structure of the zeolite is destroyed.

Heating of the boron-containing catalyst subsequent to preparation and prior to use is also preferred. The heating can be carried out in the presence of oxygen, for example, air. Heating can be at a temperature of 150°C. However, higher temperatures, e.g., up to 500°C, are preferred. Heating is generally carried out for 3—5 hours but may be extended to 24 hours or longer. While heating temperatures above 500°C can be employed, they are generally not necessary. At temperatures of 1000°C the crystal structure of the zeolite tends to deteriorate.

The amount of boron incorporated with the zeolite should be at least 0.25 percent by weight. However, it is preferred that the amount of boron in the zeolite be at least 1 percent by weight when the same is combined with a binder, e.g. 35 weight percent of alumina. The amount of boron can be as high as 20 percent by weight or more depending on the amount and type of binder present. Preferably, the amount of boron added to the zeolite is between 1.5 and 10 percent by weight. Without being limited by any theoretical considerations, it is contemplated that boron is actually present in the zeolite in an oxidized state, such as $B_2O_3$.

Antimony oxide may also be employed as a modifying component. The antimony oxide is present

as $Sb_2O_3$ alone or in admixture with other antimony oxides with or without metallic antimony or other antimony compounds being present. In all instances, regardless of the particular state of oxidation of the antimony, its content with respect to the zeolite is computed as if it were present as $Sb_2O_3$. Generally the amount of $Sb_2O_3$ in the composite catalyst will be between 6 and 40 weight percent and preferably between 10 and 35 weight percent. Antimony derivatives which may be used include: the hydride $SbH_3$; the halides $SbX_3$, $SbX_5$ (X=F, Cl, Br, I); organic alkyl and aryl stibines and their oxides $R_3Sb$, $R_5Sb$, $R_xSb{=}O$ (R=alkyl or aryl); halogen derivatives $RSbX_2$, $R_2SbX$, $RSbX_4$, $R_2SbX_3$, $R_3SbX_2$, $R_4SbX$, the acids $H_3SbO_3$, $HSbO_2$, $HSb(OH)_6$; organic acids such as $RSbO(OH)_2$, $R_2SbO \cdot OH$, all with R and X defined as above noted. Also included are organic ethers such as $R_2SbOSbR_2$; esters and alcoholates such as $Sb(OOCCH_3)_3$, $Sb(OC_4H_9)_3$, $Sb(OC_2H_5)_3$, $Sb(OCH_3)_3$; and antimonyl salts as $(SbO)SO_4$, $(SbO)NO_3$, $K(SbO)C_4H_4O_6$, $NaSbO_2 \cdot 3H_2O$.

In some instances, it may be desirable to modify the crystalline zeolite by combining therewith two or more of the specified oxides. Thus, the zeolite may be modified by prior combination therewith of oxides of phosphorus and boron, oxides of phosphorus and magnesium or oxides of magnesium and boron. When such modification technique is employed, the oxides may be deposited on the zeolite either sequentially or from a solution containing suitable compounds of the elements, the oxides of which are to be combined with the zeolite. The amounts of oxides present in such instance are in the same range as specified above for the individual oxides, with the overall added oxide content being between 0.5 and about 40 weight percent.

Still another modifying treatment entails steaming of the zeolite by contact with an atmosphere containing from 5 to 100 percent steam at a temperature of from 250° to 1000°C for a period of between 0.25 and 100 hours and under pressures ranging from sub-atmospheric to several hundred atmospheres to reduce the alpha value thereof to less than 500, and preferably less than 20, but greater than zero.

Another modifying treatment involves precoking of the catalyst to deposit a coating of between 2 and 75 and preferably between 15 and 75 weight percent of coke thereon. Precoking can be accomplished by contacting the catalyst with a hydrocarbon charge, e.g. toluene, under high severity conditions or alternatively at a reduced hydrogen to hydrocarbon concentration, i.e. 0 to 1 mole ratio of hydrogen to hydrocarbon for a sufficient time to deposit the desired amount of coke thereon.

It is also contemplated that a combination of steaming and precoking of the catalyst under the above conditions may be employed to suitably modify the crystalline zeolite catalyst.

The following examples will serve to illustrate the process of this invention without limiting the concept thereof.

Example 1

A diisopropylbenzene (DIPB) mixture containing 68.9 wt.% *meta* isomer and 23.2 wt.% *para* isomer was passed over 4.0 grams of HZSM-5 zeolite catalyst in a quartz microreactor at a feed weight hourly space velocity (WHSV) of 4.3 hr.$^{-1}$ and at temperature of 300°C to 400°C. The results are shown in Table II.

TABLE II
Selective cracking of diisopropylbenzenes

| Catalyst: HZSM-5 | Feedstock | | | |
|---|---|---|---|---|
| Temperature, °C. | — | 300 | 350 | 400 |
| WHSV, Hr$^{-1}$ | — | 4.3 | 4.3 | 4.3 |
| Composition, wt.% of aromatics | | | | |
| *meta*-DIPB | 68.9 | 73.0 | 73.0 | 72.0 |
| *para*-DIPB | 23.2 | 12.5 | 5.9 | 3.1 |
| Benzene | — | 5.2 | 9.4 | 11.7 |
| Toluene | — | 0.4 | 0.8 | 1.4 |
| $C_8$ | — | 0.8 | 1.5 | 2.3 |
| Isopropylbenzene | 0.5 | 2.6 | 1.8 | 1.3 |
| Others | 7.4 | 5.5 | 7.6 | 8.2 |
| % *meta* in DIPB | 74.8 | 85.4 | 92.5 | 95.9 |

As can be seen, at 400°C the aromatic effluent from the reactor contained 72.0 wt.% *meta*-DIPB and 3.1 wt.% *para*-DIPB. Thus the relative proporation of *meta* isomer in the DIPB has been increased from 74.8% to 95.9% by selective cracking of the *para* isomer, yielding benzene as the major cracking product.

Examples 2 and 3

In separate control experiments, the same DIPB mixture was contacted with low surface area fused quartz chips at 500°C and WHSV of less than 1 hr$^{-1}$ (Example 2) and with 4.0 grams of a

conventional amorphous silica-alumina cracking catalyst at 300°C and 380°C and WHSV of 4.3 $hr^{-1}$ (Example 3). The results are shown in Table III.

TABLE III

| | Feedstock | Example 2 | Example 3 | |
|---|---|---|---|---|
| Catalyst | — | Fused Quartz | $SiO_2 \cdot Al_2O_3$ | |
| Temperature, °C. | — | 500 | 300 | 380 |
| WHSV, $Hr^{-1}$ | — | <1 | 4.3 | 4.3 |
| Composition, wt.% of aromatics | | | | |
| *meta*-DIPB | 68.9 | 68.2 | 47.2 | 9.9 |
| *para*-DIPB | 23.2 | 23.1 | 15.4 | 3.8 |
| Benzene | — | — | 0.6 | 22.4 |
| Toluene | — | — | 0.2 | 1.3 |
| $C_8$ | — | — | 0.7 | 4.5 |
| Isopropylbenzene | 0.5 | 0.6 | 23.3 | 52.5 |
| Others | 7.4 | 8.1 | 12.6 | 5.6 |
| % *meta* in DIPB | 74.8 | 74.7 | 75.4 | 72.3 |

When the zeolite catalyst was replaced by fused quartz chips (Example 2) essentially no conversion of the DIPB occurred, even at substantially higher temperature and low feed rate thru the reactor than utilized in Example 1. This shows that an acidic catalyst is required for significant conversion of the feedstock. When the identical feedstock was brought in contact with the amorphous $SiO_2 \cdot Al_2O_3$ catalyst (Example 3), there was no enhancement of the % *meta* isomer in the DIPB feed, indicating that no selective cracking of the *para* isomer had taken place and that a shape selective catalyst is therefore required for selective destruction of the *para* isomer in preference to the *meta* isomer.

Example 4

The same mixture of *meta* and *para* DIPB was contacted with 4.0 grams of another HZSM-5 zeolite catalyst in a flow reactor at both 300°C and 315°C and at a WHSV of 4.3 $hr^{-1}$ and 19 $hr^{-1}$, respectively. The results are shown in Table IV.

TABLE IV

| Catalyst: HZSM-5 | Feedstock | | |
|---|---|---|---|
| Temperature, °C. | — | 300 | 315 |
| WHSV, $hr^{-1}$ | — | 4.3 | 19 |
| Composition, wt.% of aromatics | | | |
| *meta*-DIPB | 68.9 | 61.5 | 67.7 |
| *para*-DIPB | 23.2 | 8.2 | 12.2 |
| Benzene | — | 13.9 | 9.1 |
| Toluene | — | 1.4 | 1.0 |
| $C_8$ | — | 2.9 | 2.1 |
| Isopropylbenzene | 0.5 | 4.8 | 3.4 |
| Others | 7.4 | 7.3 | 4.5 |
| % *meta* in DIPB | 74.8 | 88.2 | 84.7 |

Even at relatively high feed rate, it can be seen that the high degree of selectivity of the ZSM-5 catalyst remains substantially unaffected.

Example 5

Preparation of Mg-P-ZSM-5 catalyst:

500 grams of $NH_4 \cdot$ZSM-5 zeolite catalyst on $Al_2O_3$ was steamed for 1 hour at 543°C, 100% steam and 1 atmosphere of pressure. The steamed catalyst was then treated with a $(NH_4)_2HPO_4$ solution for 16 hours at ambient temperature using 1.66 grams of $H_2O$, 0.568 grams $(NH_4)_2HPO_4$ per gram of catalyst. The treated catalyst was then dried at 120°C and calcined in air for 3 hours at 500°C. The phosphorus-modified zeolite was then treated with a magnesium acetate solution for 19 hours at ambient temperature using 3 grams magnesium acetate and 4 grams $H_2O$ per gram of catalyst. The treated catalyst was drained of excess solution, dried at 120°C and calcined in air at 500°C for 1 hour. The magnesium-phosphorus modified catalyst contained 2.9 wt. % Mg and 3.5 wt. % P.

Example 6

A mixture containing 52.0 wt.% meta-ethyltoluene (ET), 47.5 wt.% *para*-ET and 0.5 wt.% *ortho*-ET was contacted with 4.0 grams of the Mg-P modified catalyst of Example 5 in a flow microreactor at 400°—500°C and WHSV of 0.9—6.2 $hr^{-1}$. The results are shown in Table V.

TABLE V
Selective cracking of ethyltoluene

| Catalyst: Mg · p-ZSM-5 | Feedstock | | | |
|---|---|---|---|---|
| Temperature, °C. | — | 400 | 450 | 500 |
| WHSV, $hr^{-1}$ | — | 0.9 | 2.8 | 6.2 |
| Composition, wt.% of aromatics | | | | |
| Benzene | — | 3.1 | 2.0 | 1.5 |
| Toluene | — | 43.4 | 40.6 | 40.2 |
| Ethylbenzene | — | 3.3 | 1.8 | 0.8 |
| Dimethylbenzenes | — | 5.6 | 3.2 | 2.3 |
| *para*-Ethyltoluene | 47.5 | 9.3 | 7.3 | 5.2 |
| *meta*-Ethyltoluene | 52.0 | 32.5 | 43.1 | 49.1 |
| *ortho*-Ethyltoluene | 0.5 | 1.1 | 1.0 | 0.9 |
| Higher boiling cmpds | — | 1.7 | 1.0 | — |
| % *ortho* in Ethyltoluene | 0.5 | 2.6 | 2.0 | 1.6 |
| % *meta* in Ethyltoluene | 52.0 | 75.8 | 83.9 | 89.0 |

It is shown that the level of *meta* isomer in ethyltoluene, at 500°C and WHSV of 6.2 $hr^{-1}$, was increased from 52% in the original feed to 89% in the reactor effluent by selective cracking of the *para* isomer.

Example 7

A mixture of 26.0 wt.% *meta*-, 25.7 wt.% *para*- and 0.3 wt.% *ortho*-ethyltoluene in toluene was fed to a quartz microreactor using the same catalyst as Example 5. Conditions and results are shown in Table VI.

TABLE VI
Selective cracking of ethyltoluene in toluene

| Catalyst: Mg · P-ZSM-5 | Feedstock | | | |
|---|---|---|---|---|
| Temperature, °C. | — | 500 | 550 | 600 |
| WHSV, $hr^{-1}$ | — | 6.2 | 6.2 | 12.4 |
| Composition, wt.% of aromatics | | | | |
| Benzene | — | 1.2 | 2.5 | 2.7 |
| Toluene | 48.0 | 65.6 | 67.1 | 65.8 |
| Ethylbenzene | — | 0.6 | 0.5 | 0.3 |
| Dimethylbenzenes | — | 2.0 | 3.5 | 3.5 |
| *para*-Ethyltoluene | 25.7 | 5.3 | 2.7 | 1.9 |
| *meta*-Ethyltoluene | 26.0 | 24.9 | 23.2 | 23.8 |
| *ortho*-Ethyltoluene | 0.3 | 0.4 | 0.5 | 0.5 |
| Higher boiling cmpds | — | — | — | 1.5 |
| % *ortho* in Ethyltoluene | 0.6 | 1.3 | 1.9 | 1.9 |
| % *meta* in Ethylene | 50.0 | 81.4 | 87.9 | 90.8 |

At 500°C an increase was observed in the level of *meta* isomer from 50% in the feed to 81.4% in the reactor effluent. Similarly, the *meta* content increased to 87.9% at 550°C and 90.8% at 600°C. These results demonstrate that dilution of the reactants with a solvent does not hinder the selective cracking of the *para* isomer.

Example 8

A feedstock containing 24.05 wt.% *para*-diethylbenzene (DEB) and 25.3 wt.% *meta*-DEB with 50.4 wt.% benzene was passed over 4.0 grams of the catalyst of Example 5 at 600°C and WHSV of 12.4 $hr^{-1}$. The products are shown in Table VII.

TABLE VII
Selective cracking of diethylbenzenes

| Catalyst: Mg · P-ZSM-5 | Feedstock | |
|---|---|---|
| Temperature, °C. | — | 600 |
| WHSV, $hr^{-1}$ | — | 12.4 |
| Composition, wt.% of aromatics | | |
| Benzene | 50.4 | 61.0 |
| Toluene | — | 1.0 |
| Ethylbenzene | — | 10.0 |
| Dimethylbenzenes | — | 0.4 |
| *para*-Ethyltoluene | 0.3 | 1.4 |
| *meta*-Ethyltoluene | — | 0.5 |
| *para*-Diethylbenzene | 24.05 | 1.1 |
| *meta*-Diethylbenzene | 25.3 | 24.1 |
| *ortho*-Diethylbenzene | — | 0.6 |
| Higher boiling cmpds | — | — |
| % *meta* in Diethylbenzene | 51.3 | 93.4 |

Selective transformation of *para*-DEB has occurred, with the resultant production of benzene and ethylbenzene. The *meta*-DEB is largely unreacted. The proportion of the *meta* isomer in the DEB has increased from 51.3% in the feedstock to 93.4% in the product.

Example 9

A feedstock containing 49.5 wt.% *meta*-t-butyltoluene (t-BT) and 49.1 wt.% *para*-t-BT was contacted with 4.0 grams of HZSM-5 catalyst in a flow reactor at 420°C and WHSV of 4.4 $hr^{-1}$. The products are shown in Table VIII.

TABLE VIII
Selective cracking of t-butyltoluenes

| Catalyst: HZSM-5 | Feedstock | | |
|---|---|---|---|
| Temperature, °C. | — | 420 | 420 |
| WHSV, $hr^{-1}$ | — | 4.4 | 19.4 |
| Composition, wt.% of aromatics | | | |
| Benzene | — | 2.0 | — |
| Toluene | 0.4 | 59.5 | 26.1 |
| $C_{8, 9, 10}$ | — | 9.0 | 1.4 |
| *para*-t-Butyltoluene | 49.1 | 5.2 | 27.4 |
| *meta*-t-Butyltoluene | 49.5 | 24.2 | 44.7 |
| Higher boiling cmpds | 0.9 | 0.1 | 0.4 |
| % *meta* in t-Butyltoluene | 50.2 | 82.3 | 62.0 |

In both runs the *meta* isomer has been enriched relative to the *para* isomer, with the major conversion product being toluene.

Example 10

A mixture comprising 63.34 wt.% 1-isopropyl-3-methylbenzene (*meta*-cymene), 30.32 wt.% 1-isopropyl-4-methyl-benzene (*para*-cymene), and 2.07 wt.% 1-isopropyl-2-methylbenzene (*ortho*-cymene) was passed through a catalyst bed of 4 grams of HZSM-5 catalyst which had been steamed at 600°C for one hour with atmospheric steam. The temperature of the bed ranged between 300°C and 450°C. The results are shown in Table IX.

TABLE IX
Selective cracking of cymenes

| Catalyst: HZSM-5 | Feedstock | | |
|---|---|---|---|
| Temperature, °C. | — | 340 | 445 |
| WHSV, $hr^{-1}$ | — | 4.1 | 8.7 |
| Composition, wt.% | | | |
| Toluene | — | 20.45 | 22.96 |
| *ortho*-Cymene | 2.07 | 1.93 | 2.20 |
| *meta*-Cymene | 63.34 | 61.78 | 61.92 |

TABLE IX (contd.)

| | | | |
|---|---|---|---|
| *para*-Cymene | 30.32 | 0.0 | 0.0 |
| Aromatic $C_{10}$ | 2.15 | 5.13 | 3.16 |
| Other aromatics | 1.39 | 1.15 | 0.05 |
| $C_2H_4$ | — | 0.23 | 0.66 |
| $C_3H_6$ | — | 2.24 | 5.05 |
| $C_4H_8$ | — | 2.28 | 2.10 |
| Other light gases | 1.0 | 4.66 | 1.58 |
| % *ortho* in Cymene | 2.2 | 3.0 | 3.4 |
| % *meta* in cymene | 66.2 | 97.0 | 96.6 |

As can be seen, the *para* isomer was completely removed by catalytic cracking to lower boiling products, primarily toluene, propylene and butenes. However, the *ortho*-and-*meta*-isomers have remained practically unchanged.

Example 11

A mixture comprising 66.2 wt.% 1-isopropyl-3-methylbenzene (*meta*-cymene), 29.8 wt.% 1-isopropyl-4-methylbenzene (*para*-cymene), and 4.0 wt.% 1-isopropyl-2-methylbenzene (*ortho*-cymene) was contacted with 4 grams of HZSM-11 zeolite catalyst which had been steamed at 600°C for 3 hours at atmospheric pressure. The results are summarized in Table X.

TABLE X
Selective cracking of cymenes

| Catalyst: HZSM-11 | Feedstock | |
|---|---|---|
| Temperature, °C. | — | 310 |
| WHSV, $hr^{-1}$ | — | 4.3 |
| Composition, wt.% | | |
| Toluene | — | 36.90 |
| *ortho*-Cymene | 4.0 | 4.53 |
| *meta*-Cymene | 66.2 | 39.27 |
| *para*-Cymene | 29.8 | 1.62 |
| Aromatic $C_{10}$ | — | 3.51 |
| Other aromatics | — | 5.07 |
| $C_2H_4$ | — | 0.93 |
| $C_3H_6$ | — | 1.68 |
| $C_4H_8$ | — | 4.46 |
| Other light gases | — | 2.28 |
| % *ortho* in Cymene | 4.0 | 10.0 |
| % *meta* in Cymene | 66.2 | 86.5 |

It is again seen from the above results that the *para*-isomer has been selectively reduced with corresponding enrichment of both the *ortho* and *meta*-isomers in the cymene product fraction.

Example 12

Using a process similar to that described in Example 5, a Mg-P modified-ZSM-5 zeolite catalyst was prepared from HZSM-5 hanging 35% $Al_2O_3$ as a binder. The Mg · P-ZSM-5 contained 4.9 wt.% Mg and 3.4 wt.% P.

Example 13

A feedstock containing 47.66 wt.% *para*-ethyltoluene, 0.27 wt.% *meta*-ethyltoluene and 52.01 wt.% *ortho*-ethyltoluene was passed over 1.0 gram of the Mg · P modified ZSM-5 catalyst of Example 12 at 400°—500°C and WHSV of 3.8 $hr^{-1}$. The results are summarized in Table XI.

TABLE XI
Selective cracking of ethyltoluenes

| Catalyst: Mg · P-ZSM-5 | Feedstock | | |
|---|---|---|---|
| Temperature, °C. | — | 400 | 500 |
| WHSV, $hr^{-1}$ | | 3.8 | 3.8 |
| Composition, wt.% of aromatics | | | |
| Benzene | — | — | 0.61 |
| Toluene | — | 20.44 | 30.68 |
| Aromatic $C_8$ | — | 1.26 | 1.63 |
| *ortho*-Ethyltoluene | 52.01 | 54.19 | 56.82 |

TABLE XI (contd.)

| | | | |
|---|---|---|---|
| *meta*-Ethyltoluene | 0.27 | 3.05 | 1.38 |
| *para*-Ethyltoluene | 47.66 | 20.68 | 8.24 |
| Other $C_9^+$ Aromatics | — | 0.38 | 0.65 |
| % *ortho* in Ethyltoluene | 52.0 | 69.6 | 85.5 |
| % *meta* in Ethyltoluene | 0.3 | 3.9 | 2.1 |

Selective dealkylation of the *para*-isomer in a mixture of *ortho*- and *para*-ethyltoluene has occurred, leaving the product stream significantly enriched in the *ortho*-isomer.

Example 14

A feedstock comprising 68.13 wt.% 1-isopropyl-3-methylbenzene (*meta*-cymene), 27.54 wt.% 1-isopropyl-4-methylbenzene (*para*-cymene), and 4.25 wt.% 1-isopropyl-2-methylbenzene (*ortho*-cymene) was passed through a catalyst bed of 1.0 grams of an HZSM-23 zeolite catalyst in a flow reactor at 300°—400°C and a WHSV of 3.8 $hr^{-1}$. The runs are summarized in Table XII below.

TABLE XII
Selective cracking of cymenes

| Catalyst: HZSM-23 | Feedstock | | | |
|---|---|---|---|---|
| Temperature, °C. | — | 300 | 350 | 400 |
| WHSV, $hr^{-1}$ | — | 3.8 | 3.8 | 3.8 |
| Composition, wt.% of aromatics | | | | |
| Toluene | — | 14.2 | 30.7 | 45.5 |
| Dimethylbenzenes | — | — | — | 1.1 |
| *ortho*-Cymene | 4.25 | 4.2 | 2.9 | 1.7 |
| *meta*-Cymene | 68.13 | 64.4 | 55.3 | 38.9 |
| *para*-Cymene | 27.54 | 11.5 | 2.8 | 1.8 |
| *n*-Propyltoluene | — | — | — | 0.9 |
| % *ortho* in Cymenes | 4.3 | 5.2 | 4.8 | 4.0 |
| % *meta* in Cymenes | 68.1 | 80.4 | 90.7 | 91.8 |

As will be apparent from the data, the HZSM-23 zeolite catalyst has selectively dealkylated the *para*-isomer, leaving the product stream significantly enriched in *meta*-cymene at all temperatures.

The following examples are provided herein to illustrate the process of this invention without intending to set undue limitations thereon.

Example 15

A mixture comprising 68.2 mole % 1-hydroxy-3-isopropylbenzene and 31.5 mole % 1-hydroxy-4-isopropylbenzene was passed through a bed comprising 4 grams of HZSM-5 zeolite catalyst at a feed WHSV of 1.2. The pressure was maintained at $10^5$ N/m² (1 atm) and the temperature varied from 300°C to 400°C. The results are shown in Table XIII.

TABLE XIII

| Catalyst: HZSM-5 | Feedstock | | | |
|---|---|---|---|---|
| Temperature, °C. | — | 300 | 350 | 400 |
| WHSV | — | 1.2 | 1.2 | 1.2 |
| Liquid product composition—mole %: | | | | |
| Light ends | 0 | 2.7 | 3.8 | 5.8 |
| Hydroxybenzene | 0 | 24.9 | 38.7 | 53.3 |
| Methylhydroxybenzene | 0 | 0.5 | 0.9 | 1.8 |
| Ethylhydroxybenzene | 0 | 0.05 | 0.3 | 3.2 |
| 1-Hydroxy-2-isopropylbenzene | 0 | 1.4 | 1.3 | 0 |
| 1-Hydroxy-3-isopropylbenzene | 68.2 | 60.3 | 48.2 | 28.8 |
| 1-Hydroxy-4-isopropylbenzene | 31.5 | 9.7 | 4.5 | 2.3 |
| Other | 0 | 0.4 | 2.3 | 4.7 |
| % Hydroxyisopropylbenzene: | | | | |
| 1,2-isomer | 0 | 2.0 | 2.4 | 0 |
| 1,3-isomer | 68.2 | 84.4 | 89.2 | 92.6 |
| 1,4-isomer | 31.5 | 13.6 | 8.4 | 7.4 |
| Conversion, mole %: | | | | |
| 1,3-isomer | — | 11.6 | 29.3 | 57.8 |
| 1,4-isomer | — | 69.2 | 85.7 | 92.6 |

As will be seen from the Table, particularly from the entries entitled "Conversion", the 1-hydroxy-4-isopropylbenzene was reacted in a substantially higher amount than was the 1-hydroxy-3-isopropylbenzene, thereby demonstrating the selective character of the process utilizing HZSM-5 zeolite catalyst with a mixed isomer feed stream of disubstituted aromatic compounds containing one polar and one non-polar substituent on the benzene ring. The major product formed was hydroxybenzene, indicating that the mechanism seems to be predominantly dealkylation.

Example 16.

The same hydroxyisopropylbenzene mixture of Example 15 was passed through 4 grams of calcined amorphous aluminosilicate cracking catalyst ($SiO_2/Al_2O_3$ ratio 9/1, surface area 450 $m^2/g$) for purposes of comparison with Example 15. The WHSV and pressure were the same as those of Example 15 and the reaction was studied at 300°C and 350°C. The results were summarized in Table XIV.

TABLE XIV
Cracking of hydroxyisopropylbenzene

| Catalyst: Amorphous aluminosolicate | Feedstock | | |
|---|---|---|---|
| Temperature, °C. | — | 300 | 350 |
| WHSV | — | 1.2 | 1.2 |
| Product composition area %: | | | |
| Hydroxybenzene | 0 | 27.8 | 46.8 |
| 1-Hydroxy-2-isopropylbenzene | 0 | 7.1 | 9.2 |
| 1-Hydroxy-3-isopropylbenzene | 68.2 | 41.1 | 22.8 |
| 1-Hydroxy-4-isopropylbenzene | 31.5 | 12.2 | 7.8 |
| Others* | 0.3 | 11.7 | 13.4 |
| % Hydroxyisopropylbenzene: | | | |
| 1,2-isomer | 0 | 11.8 | 23.2 |
| 1,3-isomer | 68.2 | 68.0 | 57.2 |
| 1,4-isomer | 31.5 | 20.2 | 19.6 |
| % Conversion: | | | |
| 1,3-isomer | — | 39.7 | 66.6 |
| 1,4-isomer | — | 61.3 | 75.2 |

*including light gas, hydroxymethylbenzenes, ethylhydroxybenzenes, and *t*-butylhydroxybenzene.

It is seen that, using a conventional amorphous aluminosilicate catalyst, there has been no enrichment of the 1,3-isomer in the product stream in preference to the 1,4-isomer. In addition, transalkylation and rearrangement would seem to be enhanced in view of the higher yield of the 1,2-isomer.

A composition of Examples 15 and 16 will clearly illustrate the superiority of the present invention over a similar process employing a conventional cracking catalyst.

Example 17

ZSM-5 zeolite catalyst was modified by impregnation with magnesium as follows:

$NH_4 \cdot$ ZSM-5 was pelletized and the pellets crushed and sieved. Five grams of particles in the 14—20 mesh range were soaked in an aqueous solution of magnesium acetate (10 gm. $Mg(OAc)_2 \cdot 4H_2O$ in 20 ml. distilled $H_2O$) for 16 hours at room temperature. The catalyst was then filtered, washed with a minimum amount of water and air dried. The catalyst was calcined at 500°C for 16 hours before use. Subsequent analysis showed the catalyst to contain 3.33 wt. magnesium as MgO.

Example 18

A hydroxyisopropylbenzene mixture comprising 70.0% of the 1,3-isomer and 30.0% of the 1,4-isomer, was brought into contact with 4 grams of the Mg · HZSM-5 catalyst of Example 11. Two runs were made, the first at a feed WHSV of 4 and temperature of 350°C and the second at feed WHSV of 1.2 and temperature of 440°C. The pressure was maintained at $10^5$ $N/m^2$ (1 atm) in both runs. The results are summarized in Table XV.

TABLE XV
Selective cracking of hydroxyisopropylbenzenes

| Catalyst: Mg · HZSM-5 | Feedstock | | |
|---|---|---|---|
| Temperature, °C. | — | 350 | 440 |
| WHSV | — | 4 | 1.2 |
| Product composition—area %: | | | |
| Hydroxybenzene | 0 | 10.7 | 18.8 |
| 1-Hydroxy-2-isopropylbenzene | 0 | 0 | 0 |
| 1-Hydroxy-3-isopropylbenzene | 67.6 | 67.0 | 65.3 |
| 1-Hydroxy-4-isopropylbenzene | 28.9 | 19.9 | 11.6 |
| Others* | 3.5 | 2.4 | 4.3 |
| % Hydroxyisopropylbenzene: | | | |
| 1,2-isomer | 0 | 0 | 0 |
| 1,3-isomer | 70.0 | 77.1 | 84.8 |
| 1,4-isomer | 30.0 | 22.9 | 15.1 |
| % Conversion | | | |
| 1,3-isomer | — | 0.7 | 3.4 |
| 1,4-isomer | — | 31.2 | 59.8 |

*including light gas, hydroxymethylbenzenes, ethylhydroxybenzenes, and *t*-butylhydroxybenzenes.

The 1,4-isomer has been selectively reacted and thereby removed from the feed stream with substantially higher efficiency that the 1,3-isomer of the hydroxyisopropylbenzene, again demonstrating the efficacy of the invention. Upon the comparison of the results of this example with those of Example 15 it can be seen that the selectivity of the catalyst for cracking the 1,4-isomer has been improved by modification thereof with magnesium. In like manner it can be shown that addition of phosphorous to the catalyst will achieve similar beneficial result.

Example 19

A mixture comprising 4.8% of 2-methylbenzaldehyde, 62.4% of 3-methylbenzaldehyde and 31.2% of 4-methylbenzaldehyde was passed over 4 grams of HZSM-5 zeolite catalyst at 350°C. The feed WHSV was 1 and the pressure was $10^5$ $N/m^2$ (1 atm). The run is summarized in Table XVI below.

TABLE XVI
Selective cracking of methylbenzaldehydes

| Catalyst: HZSM-5 | Feedstock | |
|---|---|---|
| Temperature, °C. | — | 350 |
| WHSV | — | 1 |
| Liquid product composition—area % | | |
| Methylbenzene | 0 | 28.5 |
| 2-Methylbenzaldehyde | 4.8 | 4.2 |
| 3-Methylbenzaldehyde | 62.4 | 60.4 |
| 4-Methylbenzaldehyde | 31.2 | 0.9 |
| Others* | 1.7 | 6.0 |
| % Methylbenzaldehydes: | | |
| 2-Methyl isomer | 4.8 | 6.4 |
| 3-Methyl isomer | 63.4 | 92.1 |
| 4-Methyl isomer | 31.7 | 7.8 |
| % Conversion | | |
| 2-Methyl isomer | — | 11.3 |
| 3-Methyl isomer | — | 3.1 |
| 4-Methyl isomer | — | 97.0 |

*includes light gas, benzene and dimethylbenzenes

It is shown that the 4-methylbenzaldehyde has been selectively cracked in preference to both the 2-methylbenzaldehyde and the 3-methylbenzaldehyde. The major product being methylbenzene, it would appear that the reaction mechanism involved is primarily one of selective decarboxylation.

Example 20

A similar mixture of methylbenzaldehydes to that used in Example 19 was passed thru the amorphous aluminosilicate cracking catalyst of Example 16 under the same conditions of temperature, pressure and feed rate. The results are summarized in Table XVII.

TABLE XVII
Cracking of methylbenzaldehydes

| Catalyst: Amorphous aluminosilicate | Feedstock | |
|---|---|---|
| Temperature, °C. | — | 350 |
| WHSV | — | 1 |
| Liquid product composition-area %: | | |
| Methylbenzene | 0 | 6.3 |
| 2-Methylbenzaldehyde | 4.1 | 3.6 |
| 3-Methylbenzaldehyde | 64.4 | 56.8 |
| 4-Methylbenzaldehyde | 31.1 | 26.0 |
| Others* | 0.4 | 7.3 |
| % Methylbenzaldehydes: | | |
| 2-Methyl isomer | 4.1 | 4.2 |
| 3-Methyl isomer | 64.7 | 65.7 |
| 4-Methyl isomer | 31.2 | 30.1 |
| % Conversion: | | |
| 2-Methyl isomer | — | 11.3 |
| 3-Methyl isomer | — | 11.9 |
| 4-Methyl isomer | — | 16.3 |

*includes light gas, benzene and dimethylbenzenes

Using a conventional hydrocarbon cracking catalyst, no significant selectivity for preferential cracking of any isomer was observed.

Example 21

An isomeric mixture of chloroisopropylbenzenes, comprising 39.9% 1-chloro-2-isopropylbenzene, 7.1% 1-chloro-3-isopropylbenzene, and 60.0% 1-chloro-4-isopropylbenzene was passed thru 4 grams of HZSM-5 zeolite catalyst at 300°C, feed WHSV of 1.8 and pressure of $10^5$ N/m$^2$ (1 atm). Table XVIII below is a summary of the run.

TABLE XVIII
Selective cracking of chloroisopropylbenzenes

| Catalyst: HZSM-5 | Feedstock | |
|---|---|---|
| Temperature, 0°C. | — | 300 |
| WHSV | — | 1.8 |
| Product composition—area %: | | |
| Chlorobenzene | 0 | 31.0 |
| Isopropylbenzene | 0 | 0 |
| 1-Chloro-2-isopropylbenzene | 39.9 | 41.8 |
| 1-Chloro-3-isopropylbenzene | 7.1 | 7.5 |
| 1-Chloro-4-isopropylbenzene | 60.0 | 0 |
| Others* | 0 | 19.6 |
| % Chloroisopropylbenzenes: | | |
| 1,2-isomer | 39.9 | 84.7 |
| 1,3-isomer | 7.1 | 15.3 |
| 1,4-isomer | 60.0 | 0 |
| % Conversion: | | |
| 1,2-isomer | — | (—4.8) |
| 1,3-isomer | — | (—5.9) |
| 1,4-isomer | — | 100 |

*includes benzene, methylbenzene, ethylbenzene, dimethylbenzenes, chloromethylbenzenes, chloroethylbenzenes and $C_{10+}$ aromatic compounds.

The 1,4-isomer has been completely reacted, the major product being dealkylation to chlorobenzene. Some transalkylation may have taken place as indicated by the small net increase in the amount of the 1,2- and 1,3-isomer present in the product stream as compared to the feedstream.

Example 22

A feedstream comprising 55.3% 1-amino-2-isopropylbenzene and 30.7% 1-amino-4-isopropylbenzene was contacted with 4 grams of HZSM-5 zeolite catalyst at 350°C. The feed WHSV was 1 and the pressure was $10^5$ N/m$^2$ (1 atm). The results are summarized in Table XIX below.

TABLE XIX
Selective cracking of aminoisopropylbenzenes

| | Feedstock | |
|---|---|---|
| Catalyst: HZSM-5 | | |
| Temperature, °C. | — | 350 |
| WHSV | — | 1 |
| Liquid product composition—area %: | | |
| Aminobenzene | 1.2 | 12.8 |
| 1-Amino-2-isopropylbenzene | 60.3 | 58.1 |
| 1-Amino-4-isopropylbenzene | 30.7 | 22.0 |
| Others* | 7.8 | 7.1 |
| Gaseous product composition—area %: | | |
| Air | — | 15.5 |
| $C_3H_6$ | — | 77.9 |
| Other hydrocarbon gases | — | 6.6 |
| % Conversion: | | |
| 1,2-isomer | — | 3.7 |
| 1,4-isomer | — | 28.5 |

*includes unidentified aromatic compounds

The 1-amino-4-isopropylbenzene has been selectively reacted in significant preference to the 1-amino-2-isopropyl isomer, the primary products being aminobenzene and propylene.

Example 23.

The ammonium form of ZSM-11 zeolite catalyst was pelletized, crushed and sieved to 14/20 mesh particles. These were then calcined in air at 500°C for approximately 3 hours to convert the ammonium form of the catalyst into the hydrogen form. The catalyst was then selectivated by toluene disproportionation at 600°C for 3 hours to deposit coke thereon.

Example 24

A mixture comprising 69.4% 1-hydroxy-3-isopropylbenzene and 30.7% 1-hydroxy-4-isopropylbenzene was passed thru a bed of the HZSM-11 catalyst of Example 9 at 300°C and $10^5$ N/m² (1 atm). Two runs were made, one at a feed WHSV of 1.7 and the other at WHSV of 3.2. The results are summarized in TABLE XX below.

TABLE XX
Selective cracking of hydroxy isopropylbenzenes

| | Feedstock | | |
|---|---|---|---|
| Catalyst: HZSM-11 | | | |
| Temperature, °C. | — | 300 | 300 |
| WHSV | — | 1.7 | 3.2 |
| Product composition—area %: | | | |
| Hydroxybenzene | — | 41.9 | 30.9 |
| 1-Hydroxy-2-isopropylbenzene | — | 2.9 | 1.1 |
| 1-Hydroxy-3-isopropylbenzene | 69.4 | 44.0 | 54.6 |
| 1-Hydroxy-4-isopropylbenzene | 30.6 | 4.1 | 3.5 |
| Others | 0 | 7.2 | 9.9 |
| % Hydroxyisopropylbenzenes: | | | |
| 1,2-isomer | 0 | 5.5 | 1.9 |
| 1,3-isomer | 69.4 | 86.5 | 92.2 |
| 1,4-isomer | 30.6 | 8.1 | 5.9 |
| % Conversion: | | | |
| 1,3-isomer | — | 36.7 | 21.3 |
| 1,4-isomer | — | 86.6 | 88.5 |

The HZSM-11 zeolite catalyst shows substantial shape selectivity. the 1,4-isomer of hydroxyisopropylbenzene was converted to hydroxybenzene with significantly higher efficiency than was the 1,3-isomer.

Example 25

A similar hydroxyisopropylbenzene feed mixture to that of Example 10 was passed thru a bed comprising 1 gram of the zeolite catalyst HZSM-23. Two runs were made, at 300°C and 400°C

respectively, both at a feed WHSV of 6.7 and pressure of $10^5$ N/m$^2$ (1 atm). The runs are summarized in Table XXI.

TABLE XXI
Selective cracking of hydroxyisopropylbenzenes

| Catalyst: HZSM-23 | Feedstock | | |
|---|---|---|---|
| Temperature, °C. | — | 300 | 400 |
| WHSV | — | 6.7 | 6.7 |
| Product composition—area %: | | | |
| Hydroxybenzene | 0 | 2.4 | 21.6 |
| 1-Hydroxy-2-isopropylbenzene | 0 | 0.6 | 2.3 |
| 1-Hydroxy-3-isopropylbenzene | 69.4 | 73.7 | 60.9 |
| 1-Hydroxy-4-isopropylbenzene | 30.6 | 21.8 | 11.7 |
| Others | 0 | 1.5 | 3.6 |
| % Hydroxyisopropylbenzenes: | | | |
| 1,2-isomer | 0 | 0.6 | 2.9 |
| 1,3-isomer | 69.4 | 76.7 | 81.5 |
| 1,4-isomer | 30.6 | 22.7 | 15.6 |
| % Conversion: | | | |
| 1,3-isomer | — | (−6.2) | 12.3 |
| 1,4-isomer | — | 28.8 | 61.9 |

As previously demonstrated for the HZSM-11 zeolite catalysts, the zeolite catalyst designated ZSM-23 is shown to have significant shape selectivity in the preferential cracking of polar disubstituted benzenes.

Example 26

A solution comprising 29.3% of 2-chlorobenzaldehyde and 28.5% 4-chlorobenzaldehyde in benzene was passed thru a catalyst bed comprising 4 grams of HZSM-5 catalyst. Runs were carried out at both 300°C and 400°C, the feed WHSV and pressure in both cases being maintained at 2.1 and $10^5$ N/m$^2$ (1 atm) respectively. The results are summarized in Table XXII.

TABLE XXII
Selective cracking of chlorobenzaldehydes

| Catalyst: HZSM-5 | Feedstock | | |
|---|---|---|---|
| Temperature, °C. | — | 300 | 400 |
| WHSV | — | 2.1 | 2.1 |
| Product composition—area % | | | |
| Benzene | 41.8 | 42.4 | 42.9 |
| Chlorobenzene | 0 | 23.6 | 28.9 |
| 2-Chlorobenzaldehyde | 29.3 | 23.6 | 23.1 |
| 3-Chlorobenzaldehyde | 0 | 0.04 | 0.1 |
| 4-Chlorobenzaldehyde | 28.5 | 9.2 | 3.9 |
| Others | 0.4 | 1.1 | 1.1 |
| % Chlorobenzaldehyde: | | | |
| 1,2-isomer | 50.6 | 71.9 | 85.4 |
| 1,3-isomer | 0 | 0.1 | 0.2 |
| 1,4-isomer | 49.4 | 28.1 | 14.5 |
| % Conversion; | | | |
| 1,2-isomer | — | 19.3 | 21.1 |
| 1,4-isomer | — | 67.6 | 86.2 |

The selectivity of the hereindisclosed selective cracking process is shown to extend to disubstituted benzene compounds wherein both of the substituents are polar groups. In similar manner it can be shown that the described catalysts will selectively crack 1,4-disubstituted aromatics having any two polar substituents, regardless of whether such substituents are the same or different functional moieties.

**Claims**

1. A process for selective reaction by cracking and/or transalkylation of 1,4-disubstituted benzenes containing not more than one methyl substituent in a mixture comprising one or more isomers thereof, the process comprising contacting said mixture, under conversion conditions, with a

crystalline zeolite catalyst, said catalyst characterized by a silica to alumina ratio of at least 12 and a constraint index of 1 to 12, to yield a product in which the content of said 1,4-disubstituted aromatic compounds is reduced relative to the content of said isomers in said mixture prior to said contacting.

2. A process according to claim 1 wherein said 1,4-disubstituted benzene is 1,4-diisopropylbenzene, 1-ethyl-4-methylbenzene, 1,4-diethylbenzene, 1-t-butyl-4-methylbenzene, or is 1-isopropyl-4-methylbenzene.

3. A process according to claim 1 wherein said disubstituted benzene has one polar substituent and one substantially non-polar substituent.

4. A process according to claim 1 wherein said disubstituted benzene has two polar substituents.

5. A process according to claim 3 or claim 4 wherein the polar 1,4-disubstituted benzene is in admixture with one or more other polar aromatic compounds selected from the group consisting of 1,2-disubstituted aromatic compounds having at least one polar substituent and 1,3-disubstituted aromatic compounds having at least one polar substituent, said process resulting in a substantially reduction in the amount of said polar 1,4-disubstituted compound relative to said other polar aromatic compounds.

6. A process according to any preceding claim wherein said conversion conditions include a temperature between 150°C. and 800°C. and a pressure between $10^4$ N/m$^2$ and $10^7$ N/m$^2$.

7. A process according to any preceding claim wherein said conversion conditions include a temperature between 250°C and 550°C and a pressure between $2\times10^4$ N/m$^2$ and $2.5\times10^6$ N/m$^2$.

8. A process according to any preceding claim wherein said crystalline zeolite has undergone prior modification by combining therewith between 0.5 and 40 weight percent of an oxide of phosphorus, antimony, boron and/or magnesium.

9. A process according to claim 8 wherein said crystalline zeolite has undergone prior modification by combining therewith between 1 and 25 weight percent of an oxide of phosphorus or of an oxide of magnesium.

10. A process according to any of claims 1 to 7 wherein said crystalline zeolite has undergone prior modification by steaming at a temperature between 250°C and 1000°C for between 0.5 and 100 hours.

11. A process according to any preceding claim wherein said crystalline zeolite is ZSM-5, ZSM-11 or ZSM-23.

12. A process according to any preceding claim wherein said throttle is admixed with a binder.

13. A process according to any preceding claim wherein said mixture containing said 1,4-disubstituted benzene is admixed with a diluent prior to contacting it with said zeolite catalyst.

**Patentansprüche**

1. Verfahren zur selektiven Reaktion unter Cracken und/oder Transalkylierung von 1,4-disubstituierten Benzolen, die nicht mehr als einen Methylsubstituenten aufweisen, in einer Mischung, die eines oder mehrere Isomere davon enthält, wobei bei dem Verfahren die genannte Mischung unter Umwandlungsbedingungen mit einem kristallinen Zeolith-Katalysator in Kontakt gebracht wird, wobei dieser Katalysator durch ein Verhältnis von Siliciumoxid zu Aluminiumoxid von wenigstens 12 und einen Grenzwertindex (constraint index) von 1 bis 12 gekennzeichnet ist, und wobei ein Produkt erhalten wird, bei dem der Gehalt an den genannten 1,4-disubstituierten aromatischen Verbindung vermindert ist, verglichen mit dem Gehalt der genannten Isomeren in der genannten Mischung vor dem genannten Inkontaktbringen.

2. Verfahren nach Anspruch 1, bei dem das genannte 1,4-disubstituierte Benzol 1,4-Diisopropylbenzol, 1-Ethyl-4-methylbenzol, 1,4-Diethylbenzol, 1-t-Butyl-4-methylbenzol oder 1-Isopropyl-4-methylbenzol ist.

3. Verfahren nach Anspruch 1, bei dem des genannte disubstituierte Benzol einen polaren Substituenten und einen im wesentlichen unpolaren Substituenten aufweist.

4. Verfahren nach Anspruch 1, bei dem das genannte disubstituierte Benzol 2 polare Substituenten aufweist.

5. Verfahren nach Anspruch 3 oder Anspruch 4, bei dem das polare 1,4-disubstituierte Benzol in einer Mischung mit einer oder mehreren anderen polaren aromatischen Verbindungen vorliegt, die aus einer Gruppe ausgewählt sind, die aus 1,2-disubstituierten aromatischen Verbindungen, die wenigstens einen polaren Substituenten aufweisen, und 1,3-disubstituierten aromatischen Verbindungen, die wenigstens einen polaren Substituenten aufweisen, besteht, wobei das Verfahren zu einer wesentlichen Verminderung der Menge der genannten polaren 1,4-disubstituierten Verbindungen gegenüber den genannten anderen polaren aromatischen Verbindungen führt.

6. Verfahren nach einem beliebigen, er vorangehenden Ansprüche, bei dem die genannten Umwandlungsbedingungen eine Temperatur zwischen 150°C und 800°C und einen Druck zwischen $10^4$ N/m$^2$ und $10^7$ N/m$^2$ beinhalten.

7. Verfahren nach einem beliebigen der vorangehenden Ansprüche, bei dem die genannten Umwandlungsbedingungen eine Temperatur zwischen 250°C und 550°C und einen Druck zwischen $2\times10^4$ N/m$^2$ und $2{,}5\times10^6$ N/m$^2$ beinhalten.

8. Verfahren nach einem beliebigen der vorangehenden Ansprüche bei dem der genannte kristalline Zeolith einer vorausgehenden Modifizierung unterzogen wurde, indem er mit zwischen 0,5 und 40 Gew.-% eines Oxids von Phosphor, Antimon, Bor und/oder Magnesium kombiniert wurde.

9. Verfahren nach Anspruch 8, bei dem der genannte kristalline Zeolith einer vorausgehenden Modifizierung unterzogen wurde, indem er mit zwischen 1 und 25 Gew.-% eines Oxids von Phosphor oder eines Oxids von Magnesium kombiniert wurde.

10. Verfahren nach einem der Ansprüche 1 bis 7, bei dem der genannte kristalline Zeolith einer vorausgehenden Modifizierung unterzogen wurde, indem er bei einer Temperatur zwischen 250°C und 1000°C für zwischen 0,5 und 100 Stunden dampfbehandelt wurde.

11. Verfahren nach einem beliebigen der vorangehenden Ansprüche, bei dem der genannte kristalline Zeolith ZSM-5, ZSM-11 oder ZSM-23 ist.

12. Verfahren nach einem beliebigen der vorangehenden Ansprüche, bei dem der genannte Zeolith mit einem Bindemittel vermischt ist.

13. Verfahren nach einem beliebigen der vorangehenden Ansprüche, bei dem die genannte Mischung, die das genannte 1,4-disubstituierte Benzol enthält, vor dem Inkontaktbringen mit dem genannten Zeolith-Katalysator mit einem Verdünnungsmittel vermischt wird.

**Revendications**

1. Un procédé pour la réaction sélective par craquage et/ou transalkylation de benzènes disubstitués en 1,4 ne contenant pas plus d'un substituant méhyle dans un mélange comprenant un ou plusieurs de leurs isomères, le procédé comprenant le contact dudit mélange, dans des conditions de conversion, avec un catalyseur constitué de zéolite cristalline, ledit catalyseur étant caractérisé par un rapport de la silice à l'alumine d'au moins 12 et un indice de contrainte et 1 à 12, pour fournir un produit dans lequel la teneur desdits composés aromatiques disubstitués en 1,4 est réduite par rapport à la teneur desdits isomères dudit mélange avant ledit contact.

2. Un procédé selon la revendication 1, dans lequel ledit benzène disubstitué en 1,4 est le diisopropyl-1,4 benzène, l-éthyl-1 méthyl-4 benzène, le diéthyl-2,4 benzène, le tert-butyl-1 méthyl-4 benzène ou l'isopropyl-1 méthyl-4 benzène.

3. Un procédé selon la revendication 1, dans lequel ledit benzène disubstitué a un substituant polaire et un substituant essentiellement non polaire.

4. Un procédé selon la revendication 1, dans lequel ledit benzène disubstitué a deux substituants polaires.

5. Un procédé selon l'une des revendications 3 ou 4, dans lequel le benzène disubstitué en 1,4 polaire est en mélange avec un ou plusieurs autres composés aromatiques polaires choisis parmi le groupe constitué par les composés aromatiques disubstitués en 1,2 ayant au moins un substituant polaire et les composés aromatiques disubstitués en 1,3 ayant au moins un substituant polaire, ledit procédé produisant une réduction notable de la quantité dudit composé disubstitué en 1,4 polaire par rapport auxdits autres composés aromatiques polaires.

6. Un procédé selon l'une quelconque des revendications précédentes, dans lequel lesdites conditions de conversion comprennant une température entre 150 et 800°C et une pression entre $10^4$ N/m$^2$ et $10^7$ N/m$^2$.

7. Un procédé selon l'une quelconque des revendications précédentes, dans lequel lesdites conditions de conversion comprennent une température entre 250°C et 550°C et une pression entre $2\times10^4$ N/m$^2$ et $2{,}5\times10^6$ N/m$^2$.

8. Un procédé selon l'une quelconque des revendications précédentes dans lequel ladite zéolite cristalline a subi une modification préable par combinaison avec entre 0,5 et 40% en poids d'un oxyde de phosphore, d'antimoine, de bore et/ou de magnésium.

9. Un procédé selon la revendication 8, dans lequel ladite zéolite cristalline a subi une modification préalable par combinaison avec entre 1 et 25% en poids d'un oxyde de phosphore et/ou d'un oxyde de magnesium.

10. Un procédé selon l'une quelconque des revendications 1 à 7, dans lequel ladite zéolite cristalline a subi une modification préalable par traitement par la vapeur d'eau à une température entre 250°C et 1000°C, pendant une durée de 0,5 à 100 h.

11. Un procédé selon l'une quelconque des revendications précédentes, dans lequel ladite zéolite cristalline est la ZSM-5, la ZSM-11 ou la ZSM-23.

12. Un procédé selon l'une quelconque des revendications précédentes, dans lequel ladite zéolite est mélangée avec un liant.

13. Un procédé selon l'une quelconque des revendications précédentes, dans lequel ledit mélange contenant ledit benzène disubstitué en 1,4 est mélangé avec un diluant avant son contact avec ledit catalyseur zéolitique.